# EUROPEAN PATENT SPECIFICATION

(11) **EP 3 357 503 B1**
(45) Date of publication and mention of the grant of the patent: **30.10.2019**
(21) Application number: 18162214.3
(22) Date of filing: 21.01.2011
(51) Int. Cl.: A61K 38/18, A61K 38/26, A61P 3/00

(54) **PHARMACEUTICAL COMPOSITION FOR TREATING A METABOLIC SYNDROME**
PHARMAZEUTISCHE ZUSAMMENSETZUNG ZUR BEHANDLUNG EINES STOFFWECHSELSYNDROMS
COMPOSITION PHARMACEUTIQUE DE TRAITEMENT D'UN SYNDROME MÉTABOLIQUE

(30) Priority: 21.01.2010 EP 10305070
(43) Date of publication of application: 08.08.2018
(62) Divisional of application: 16205881.2
(73) Proprietor: Sanofi, 75008 Paris (FR)
(72) Inventor: Sommerfeld, Mark, 65926 Frankfurt am Main (DE); Schaefer, Hans-Ludwig, 65926 Frankfurt am Main (DE); Boscheinen, Oliver, 65926 Frankfurt am Main (DE); Habermann, Paul, deceased (DE); Rao, Ercole, 65926 Frankfurt am Main (DE); Dreyer, Matthias, 65926 Frankfurt am Main (DE)
(74) Representative: Böhmer, Thomas

(56) References cited:
- WO-A1-98/19698
- WO-A1-2010/142665
- WO-A2-03/011213
- WO-A2-2009/020802
- JP-A- 2002 112 772
- US-A1- 2008 255 045
- WENTE WOLF ET AL: "Fibroblast growth factor-21 improves pancreatic beta-cell function and survival by activation of extracellular signal-regulated kinase 1/2 and Akt signaling pathways.", September 2006 (2006-09), DIABETES SEP 2006 LNKD- PUBMED:16936195, VOL. 55, NR. 9, PAGE(S) 2470 - 2478, XP002584862, ISSN: 0012-1797 * abstract *

## Description

The present invention is directed to a pharmaceutical composition containing at least one FGF-21 (fibroblast growth factor 21) compound, at least one GLP-1 R (glucagon-like peptide-1 receptor) agonist and optionally at least one anti-diabetic drug and/or at least one DPP-4 (dipeptidyl peptidase-4) inhibitor for use in the treatment of Type 2-diabetes in a patient, wherein the patient is a sulfonylurea-treated Type 2-diabetic patient.

Diabetes mellitus is characterized by its clinical manifestations, namely the non-insulin-dependent or maturity onset form, also known as Type 2 diabetes and the insulin-dependent or juvenile onset form, also known as Type 1 diabetes. The manifestations of clinical symptoms of Type 2 diabetes and the underlying obesity usually appear at an age over 40. In contrast, Type 1 diabetes usually shows a rapid onset of the disease often before 30. The disease is a metabolic disorder in humans with a prevalence of approximately one percent in the general population, with one-fourth of these being Type 1 and three-fourth of these being Type 2 diabetes. Type 2 diabetes is a disease characterized by high-circulating blood glucose, insulin and corticosteroid levels.

Currently, there are various pharmacological approaches for the treatment of Type 2 diabetes, which may be utilized individually or in combination, and which act via different modes of action:
1) sulfonylurea stimulate insulin secretion;
2) biguanides (metformin) act by promoting glucose utilization, reducing hepatic glucose production and diminishing intestinal glucose output;
3) oc-glucosidase inhibitors (acarbose, miglitol) slow down carbohydrate digestion and consequently absorption from the gut and reduce postprandial hyperglycemia;
4) thiazolidinediones (troglitazone) enhance insulin action, thus promoting glucose utilization in peripheral tissues; and
5) insulin stimulates tissue glucose utilization and inhibits hepatic glucose output.

However, most of the drugs have limited efficacy and do not address the most important problems, the declining β-cell function and the associated obesity.

Obesity is a chronic disease that is highly prevalent in modern society and is associated with numerous medical problems including diabetes mellitus, insulin resistance, hypertension, hypercholesterolemia, and coronary heart disease. It is further highly correlated with diabetes and insulin resistance, the latter of which is generally accompanied by hyperinsulinemia or hyperglycemia, or both. In addition, Type 2 diabetes is associated with a two to fourfold risk of coronary artery disease.

Type 1 diabetics characteristically show very low or immeasurable plasma insulin with elevated glucagon. An immune response specifically directed against β-cells leads to Type 1 diabetes because β-cells secrete insulin.

Current therapeutic regimens for Type 1 diabetes try to minimize hyperglycemia resulting from the lack of natural insulin.

Fibroblast growth factor 21 (FGF21) is a novel metabolic regulator produced primarily by the liver that exerts potent antidiabetic and lipid-lowering effects in animal models of obesity and type 2 diabetes mellitus. This hormone contributes to body weight regulation and is involved in the response to nutritional deprivation and ketogenic state in mice. The principal sites of metabolic actions of FGF21 are adipose tissue, liver and pancreas. Experimental studies have shown improvements in diabetes compensation and dyslipidemia after FGF21 administration in diabetic mice and primates (Dostalova *et al.* 2009). FGF21 has been shown to stimulate glucose uptake in mouse 3T3-L1 adipocytes in the presence and absence of insulin, and to decrease fed and fasting blood glucose, triglycerides, and glucagon levels in ob/ob and db/db mice and 8 week old ZDF rats in a dose dependent manner, thus, providing the basis for the use of FGF-21 as a therapy for treating diabetes and obesity (see e.g. WO03/011213).

Fibroblast growth factors (FGFs) are polypeptides widely expressed in developing and adult tissues. The FGF family currently consists of twenty-two members, FGF-1 to FGF-23. The members of the FGF family are highly conserved in both gene structure and amino acid sequence between vertebrate species. There are 18 mammalian fibroblast growth factors (FGF1-FGF10 and FGF16-FGF23) which are grouped into 6 subfamilies based on differences in sequence homology and phylogeny. The numbered 'FGFs' that are unassigned to subfamilies - the FGF homologous factors (previously known as FGF11-FGF14) - have high sequence identity with the FGF family but do not activate FGF receptors (FGFRs) and are therefore not generally considered members of the FGF family.

While most of FGFs act as local regulators of cell growth and differentiation, recent studies indicated that FGF19 subfamily members including FGF15/19, FGF21 and FGF23 exert important metabolic effects by an endocrine fashion. The members of FGF19 subfamily regulate diverse physiological processes that are not affected by classical FGFs. The wide variety of metabolic activities of these endocrine factors include the regulation of the bile acid, carbohydrate and lipid metabolism as well as phosphate, calcium and vitamin D homeostasis (Tomlinson *et al.* 2002, Holt *et al.* 2003, Shimada *et al.* 2004, Kharitonenkov *et al.* 2005, Inagaki *et al.* 2005, Lundasen *et al.* 2006).

FGF21 was originally isolated from mouse embryos. FGF21 mRNA was most abundantly expressed in the liver, and to lesser extent in the thymus (Nishimura *et al.* 2000). Human FGF21 is highly identical (approximately 75 % amino acid identity) to mouse FGF21. Among human FGF family members, FGF21 is the most similar (approximately 35 % amino acid identity) to FGF19 (Nishimura *et al.* 2000). FGF21 is free of the proliferative and tumorigenic effects (Kharitonenkov *et al.* 2005, Huang *et al.* 2006, Wente *et al.* 2006) that are typical for majority of the members of FGF family (Ornitz and Itoh 2001, Nicholes *et al.* 2002, Eswarakumar *et al.* 2005).

The administration of FGF21 to obese leptin-deficient *ob*/*ob* and leptin receptor-deficient *db*/*db* mice and obese ZDF rats significantly lowered blood glucose and triglycerides, decreased fasting insulin levels and improved glucose clearance during an oral glucose tolerance test. FGF21 did not affect food intake or body weight/composition of diabetic or lean mice and rats over the course of 2 weeks of administration. Importantly, FGF21 did not induce mitogenicity, hypoglycemia, or weight gain at any dose tested in diabetic or healthy animals or when overexpressed in transgenic mice (Kharitonenkov *et al.* 2005). FGF21-overexpressing transgenic mice were resistant to diet-induced obesity.

The administration of FGF21 to diabetic rhesus monkeys for 6 weeks reduced fasting plasma glucose, fructosamine, triglyceride, insulin and glucagon levels. Importantly, hypoglycemia was not observed during the study despite of significant glucose-lowering effects. FGF21 administration also significantly lowered LDL-cholesterol and increased HDL-cholesterol and, in contrast to mice (Kharitonenkov *et al.* 2005), slightly but significantly decreased body weight (Kharitonenkov *et al.* 2007).

Further information can be taken from the following references:
1. DOSTALOVA I. et al.: Fibroblast Growth Factor 21: A Novel Metabolic Regulator With Potential Therapeutic Properties in Obesity/Type 2 Diabetes Mellitus. Physiol Res 58: 1-7, 2009.
2. ESWARAKUMAR V.P. et al.: Cellular signaling by fibroblast growth factor receptors. Cytokine Growth Factor Rev 16: 139-149, 2005.
3. HOLT J.A. et al.: Definition of a novel growth factor-dependent signal cascade for the suppression of bile acid biosynthesis. Genes Dev 17: 1581-1591, 2003.
4. HUANG X. et al.: Forced expression of hepatocytespecific fibroblast growth factor 21 delays initiation of chemically induced hepatocarcinogenesis. Mol Carcinog 45: 934-942, 2006.
5. INAGAKI T. et al.: Endocrine regulation of the fasting response by PPARα-mediated induction of fibroblast growth factor 21. Cell Metab 5: 415-425, 2007.
6. KHARITONENKOV A. et al.: FGF-21 as a novel metabolic regulator. J Clin Invest 115: 1627-1635, 2005.
7. KHARITONENKOV A. et al.: The metabolic state of diabetic monkeys is regulated by fibroblast growth factor-21. Endocrinology 148: 774-781, 2007.
8. LUNDÅSEN T. et al.: Circulating intestinal fibroblast growth factor 19 has a pronounced diurnal variation and modulates hepatic bile acid synthesis in man. J Intern Med 260: 530-536, 2006.
9. NICHOLES K. et al.: A mouse model of hepatocellular carcinoma: ectopic expression of fibroblast growth factor 19 in skeletal muscle of transgenic mice. Am J Pathol 160: 2295-2307, 2002.
10. NISHIMURA T. et al.: Identification of a novel FGF, FGF-21, preferentially expressed in the liver. Biochim Biophys Acta 1492: 203-206, 2000.
11. ORNITZ D.M. et al.: Fibroblast growth factors. Genome Biol 2: REVIEWS3005, 2001.
12. SHIMADA T. et al.: FGF-23 is a potent regulator of vitamin D metabolism and phosphate homeostasis. J Bone Miner Res 19: 429-435, 2004.
13. TOMLINSON E. et al.: Transgenic mice expressing human fibroblast growth factor-19 display increased metabolic rate and decreased adiposity. Endocrinology 143: 1741-1747, 2002.
14. WENTE W. et al.: Fibroblast growth factor-21 improves pancreatic beta-cell function and survival by activation of extracellular signal-regulated kinase 1/2 and Akt signaling pathways. Diabetes 55: 2470-2478, 2006.

The gut peptide glucagon-like peptide-1 (GLP-1) is an incretin hormone and secreted in a nutrient-dependent manner. It stimulates glucose-dependent insulin secretion. GLP-1 also promotes β-cell proliferation and controls glycemia via additional actions on glucose sensors, inhibition of gastric emptying, food intake and glucagon secretion. Furthermore, GLP-1 stimulates insulin secretion and reduces blood glucose in human subjects with Type 2 diabetes. Exogenous administration of bioactive GLP-1, GLP-1 (7-27) or GLP-1(7-36 amide), in doses elevating plasma concentrations to approximately 3-4 fold physiological postprandial levels fully normalizes fasting hyperglycaemia in Type 2 diabetic patients (Nauck, M. A. et al. (1997) Exp Clin Endocrinol Diabetes, 105, 187-197). The human GLP-1 receptor (GLP-1R) is a 463 amino acid heptahelical G protein-coupled receptor widely expressed in pancreatic islets, kidney, lung, heart and multiple regions of the peripheral and central nervous system. Within islets, the GLP-1 R is predominantly localized to islet β-cells. Activation of GLP-1R signalling initiates a program of differentiation toward a more endocrine-like phenotype, in particular the differentiation of progenitors derived from human islets into functioning β-cells (Drucker, D. J. (2006) Cell Metabolism, 3, 153-165).

Unfortunately, both, FGF-21 and bioactive GLP-1, as well as other known drugs have limited efficacy by themselves to the complex and multifactorial metabolic dysfunctions which can be observed in Type 2 diabetes or other metabolic disorders. This applies also for the efficacy in lowering the blood glucose levels by said compounds themselves.

According to the present invention it has surprisingly been found that the combination of FGF-21 and a GLP-1R agonist significantly lowered blood glucose levels in a synergistic manner up to normo-glycaemic levels.

One embodiment of the present invention is, therefore, directed to a pharmaceutical composition containing at least one FGF-21 (fibroblast growth factor 21) compound and at least one GLP-1R (glucagon-like peptide-1 receptor) agonist for use in treating Type 2-diabetes in a patient, as defined in claim 1.

A "FGF-21 compound" is defined as a compound showing FGF-21 activity, in particular comprising (i) native FGF-21, especially human FGF-21, in particular human FGF-21 as shown in SEQ ID NO: 1, or (ii) a FGF-21 mimetic with FGF-21 activity.

"FGF-21 activity" is usually measured in a FGF-21 activity assay generally known to a person skilled in the art. An FGF-21 activity assay is e.g. a "glucose uptake assay" as described in Kharitonenkov, A. et al. (2005), 115; 1627, No. 6. As an example for the glucose uptake assay, adipocytes are starved for 3 hours in DMEM/0.1% BSA, stimulated with FGF-21 for 24 hours, and washed twice with KRP buffer (15 mM HEPES, pH 7.4, 118 mM NaCl, 4.8 mM KCl, 1.2 mM MgSO₄, 1.3 mM CaCl₂, 1.2 mM KH₂PO₄, 0.1% BSA), and 100 µl of KRP buffer containing 2-deoxy-D-[¹⁴C]glucose (2-DOG) (0.1 µCi 100 µM) is added to each well. Control wells contains 100 µl of KRP buffer with 2-DOG (0.1 µCi, 10 mM) to monitor for nonspecificity. The uptake reaction is carried out for 1 hour at 37°C, terminated by addition of cytochalasin B (20 µM), and measured using Wallac 1450 MicroBeta counter (PerkinElmer, USA).

Examples of FGF-21 mimetics are (a) proteins having at least about 96%, in particular 99% amino acid sequence identity to the amino acid sequence shown in SEQ ID NO: 1 and having FGF-21 activity, (b) a FGF-21 fusion protein or a (c) FGF-21 conjugate, e.g. a FGF-21 mutein, a FGF-21-Fc fusion protein, a FGF-21-HSA fusion protein or a PEGylated FGF-21.

Examples of FGF-21 muteins are described in e.g. WO2005/061712, WO2006/028595, WO2006/028714, WO2006/065582 or WO2008/121563. Exemplary muteins are muteins which have a reduced capacity for O-glycosylation when e.g. expressed in yeast compared to wild-type human FGF-21, e.g. human FGF-21 with a substitution at position 167 (serine), e.g. human FGF-21 with one of the following substitutions: Ser167Ala, Ser167Glu, Ser167Asp, Ser167Asn, Ser167Gln, Ser167Gly, Ser167Val, Ser167His, Ser167Lys or Ser167Tyr. Another example is a mutein which shows reduced deamidation compared to wild-type human FGF-21, e.g. a mutein with a substitution at position 121 (asparagine) of human FGF-21, e.g. Asn121Ala, Asn121Val, Asn121Ser, Asn121Asp or Asn121Glu. An alternative mutein is human FGF-21 having one or more non-naturally encoded amino acids, e.g. as described by the general formula in claim 29 of WO2008/121563. Other muteins comprise a substitution of a charged (e.g. aspartate, glutamate) or polar but uncharged amino acid (e.g. serine, threonine, asparagine, glutamine) for e.g. a polar but uncharged or charged amino acid, respectively. Examples are Leu139Glu, Ala145Glu, Leu146Glu, Ile152Glu, Gln156Glu, Ser163Glu, Ile152Glu, Ser163Glu or Gln54Glu. Another mutein is a mutein showing a reduced susceptibility for proteolytic degradation when expressed in e.g. yeast compared to human FGF-21, in particular human FGF-21 with a substitution of Leu153 with an amino acid selected from Gly, Ala, Val, Pro, Phe, Tyr, Trp, Ser, Thr, Asn, Asp, Gln, Glu, Cys or Met. A preferred FGF-21 mutein is the mutated FGF-21 according to SEQ ID NO: 2 which carries a deletion of amino acids 1-28 of human FGF-21 (SEQ ID NO: 1) and contains an additional glycine at the N-terminus.

Examples of FGF-21 fusion proteins are described in e.g. WO2004/110472 or WO2005/113606, for example a FGF-21-Fc fusion protein or a FGF-21-HSA fusion protein. "Fc" means the Fc portion of an immunoglobulin, e.g. the Fc portion of IgG4. "HSA" means human serum albumin.

Examples of FGF-21 conjugates are described in e.g. WO2005/091944, WO2006/050247 or WO2009/089396, for example glycol-linked FGF-21 compounds. Such glycol-linked FGF21 compounds usually carry a polyethylene glycol (PEG), e.g. at a cysteine or lysine amino acid residue or at an introduced N-linked or O-linked glycosylation site, (herein referred to as "PEGylated FGF-21"). Such PEGylated FGF-21 compounds generally show an extended time of action compared to human FGF-21. Suitable PEGs have a molecular weight of about 20,000 to 40,000 daltons.

A "GLP-1R agonist" is defined as a compound which binds to and activates the GLP-1 receptor, like GLP-1 (glucagon-like peptide 1). Physiological actions of GLP-1 and/or of the GLP-1R agonist are described e.g. in Nauck, M. A. et al. (1997) Exp. Clin. Endocrinol. Diabetes, 105, 187-195. These physiological actions in normal subjects, in particular humans, include e.g. glucose-dependent stimulation of insulin secretion, suppression of glucagon secretion, stimulation of (pro)insulin biosynthesis, reduction of food intake, deceleration of gastric emptying and/or equivocal insulin sensitivity.

Suitable assays to discover GLP-1 R agonists are described in e.g. Thorkildsen, Chr. et al. (2003), Journal of Pharmacology and Experimental Therapeutics, 307, 490-496; Knudsen, L. B. et al. (2007), PNAS, 104, 937-942, No. 3; Chen, D. et al. (2007), PNAS, 104, 943-948, No. 3; or US2006/0003417 A1 (see e.g. Example 8). In short, in a "receptor binding assay", a purified membrane fraction of eukaryotic cells harbouring e.g. the human recombinant GLP-1 receptor, e.g. CHO, BHK or HEK293 cells, is incubated with the test compound or compounds in the presence of e.g. human GLP-1, e.g. GLP-1 (7-36) amide which is marked with e.g. ¹²⁵I (e.g. 80 kBq/pmol). Usually different concentrations of the test compound or compounds are used and the IC₅₀ values are determined as the concentrations diminishing the specific binding of human GLP-1. In a "receptor functional assay", isolated plasma membranes from eukaryotic cells, as e.g. described above, expressing e.g. the human GLP-1 receptor were prepared and incubated with a test compound. The functional assay is carried out by measuring cAMP as a response to stimulation by the test compound. In a "reporter gene assay", eukaryotic cells, as e.g. described above, expressing e.g. the human GLP-1 receptor and containing e.g. a multiple response element/cAMP response element-driven luciferase reporter plasmid are cultured in the presence of a test compound. cAMP response element-driven luciferase activities are measured as a response to stimulation by the test compound.

Suitable GLP-1R agonists are selected from a bioactive GLP-1, a GLP-1 analog or a GLP-1 substitute, as e.g. described in Drucker, D. J. (2006) Cell Metabolism, 3, 153-165; Thorkildsen, Chr. (2003; supra); Chen, D. et al. (2007; supra); Knudsen, L. B. et al. (2007; supra); Liu, J. et al. (2007) Neurochem Int., 51, 361-369, No. 6-7; Christensen, M. et al. (2009), Drugs, 12, 503-513; Maida, A. et al. (2008) Endocrinology, 149, 5670-5678, No. 11 and US2006/0003417. Exemplary compounds are GLP-1(7-37), GLP-1(7-36)amide, exendin-4, liraglutide, CJC-1131, *albugon*, *albiglutide*, exenatide, *exenatide-LAR*, oxyntomodulin, lixisenatide, geniproside, *AVE-0010*, a short peptide with GLP-1R agonistic activity and/or a small organic compound with GLP-1R agonistic activity.

In detail, human GLP-1(7-37) possesses the amino acid sequence of SEQ ID NO: 3. Human GLP-1(7-36)amide possesses the amino acid sequence of SEQ ID NO: 4. Exendin-4 possesses the amino acid sequence of SEQ ID NO: 5. Exenatide possesses the amino acid sequence of SEQ ID NO: 6 and oxyntomodulin the amino acid sequence of SEQ ID NO: 7. The amino acid sequence of lixisenatide is shown in SEQ ID NO: 8. The structure of lixisenatide is based on exendin-4(1-39) modified C-terminally with six additional lysine residues in order to resist immediate physiological degradation by DPP-4 (dipeptidyl peptidase-4). The amino acid sequence of *AVE0010* is shown in SEQ ID NO: 9.

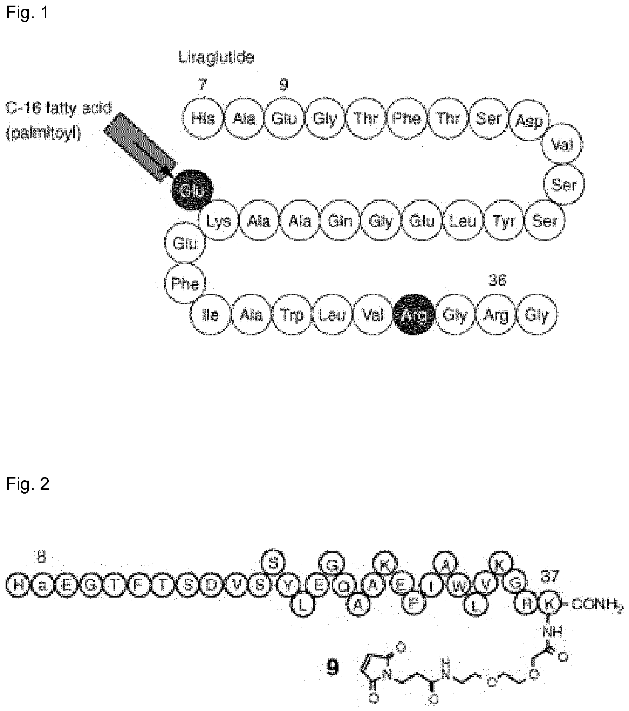

The chemical structure of liraglutide is shown in Fig. 1. Liraglutide was obtained by substitution of Lys 34 of GLP-1(7-37) to Arg, and by addition of a C16 fatty acid at position 26 using a γ-glutamic acid spacer. The chemical name is [N-epsilon(gamma-L-glutamoyl(N-alpha-hexadecanoyl)-LyS²⁶,Arg³⁴-GLP-1(7-37)].

The chemical structure of CJC-1131 is shown in Fig. 2. Albumin is attached at the *C-*terminal of GLP-1 with a d-alanine substitution at position 8. CJC-1131 shows a very good combination of stability and bioactivity.

Other peptides with GLP-1R agonistic activity are exemplary disclosed in US 2006/0003417 and small organic compound with GLP-1R agonistic activity are exemplary disclosed in Chen et al. 2007, PNAS, 104, 943-948, No. 3 or Knudsen et al., 2007, PNAS, 104, 937-942.

In a further embodiment of the present invention the pharmaceutical composition additionally comprises at least one anti-diabetic drug and/or at least one DPP-4 inhibitor.

Exemplary anti-diabetic drugs are
a) insulin,
b) thiazolidinedione, e.g. rosiglitazone or pioglitazone (see e.g. WO2005/072769), metformin (*N*,*N*-dimethylimidodicarbonimidic-diamide), or
c) sulphonylurea, such as chlorpropamide (4-chloro-*N*-(propylcarbamoyl)-benzenesulfonamide), tolazamide (*N*-[(azepan-1-ylamino)carbonyl]-4-methyl-benzenesulfonamide), gliclazide (*N*-(hexahydrocyclopenta[*c*]pyrrol-2(1*H*)-yl-carbamoyl)-4-methylbenzenesulfonamide), or glimepiride (3-ethyl-4-methyl-*N*-(4-[*N-*((1*r*,4*r*)-4-methytcyctohexylcarbamoyl)-sulfamoyl]phenethyl)-2-oxo-2,5-dihydro-1*H-*pyrrole-1-carboxamide).

According to the present invention "insulin" means naturally occurring insulin, modified insulin or an insulin analogue, including salts thereof, and combinations thereof, e.g. combinations of a modified insulin and an insulin analogue, for example insulins which have amino acid exchanges/deletions/additions as well as further modifications such as acylation or other chemical modification. One example of this type of compound is insulin detemir, i.e. LysB29-tetradecanoyl/des(B30) human insulin. Another example may be insulins in which unnatural amino acids or amino acids which are normally noncoding in eukaryotes, such as D-amino acids, have been incorporated (Geiger, R. et al., Hoppe Seylers Z. Physiol. Chem. (1976) 357, 1267-1270; Geiger, R. et al., Hoppe Seylers Z. Physiol. Chem. (1975) 356, 1635-1649, No. 10; Krail, G. et al., Hoppe Seylers Z. Physiol. Chem. (1971) 352, 1595-1598, No. 11). Yet other examples are insulin analogues in which the C-terminal carboxylic acid of either the A-chain or the B-chain, or both, are replaced by an amide.

"Modified insulin" is preferably selected from acylated insulin with insulin activity, in particular wherein one or more amino acid(s) in the A and/or B chain of insulin is/are acylated, preferably human insulin acylated at position B29 (Tsai, Y. J. et al. (1997) Journal of Pharmaceutical Sciences, 86, 1264-1268, No. 11). Other acetylated insulins are desB30 human insulin or B01 bovine insulin (Tsai, Y. J. et al., supra). Other Examples of acylated insulin are e.g. disclosed in US 5,750,497 and US 6,011,007. An overview of the structure-activity relationships for modified insulins, is provided in Mayer, J. P. et al. (2007) Biopolymers, 88, 687-713, No. 5. Modified insulins are typically prepared by chemical and/or enzymatic manipulation of insulin, or a suitable insulin precursor such as preproinsulin, proinsulin or truncated analogues thereof.

An "insulin analogue" is preferably selected from insulin with insulin activity having one or more mutation(s), substitution(s), deletion(s) and/or addition(s), in particular an insulin with a C- and/or N-terminal truncation or extension in the A and/or B chain, preferably des(B30) insulin, PheB1 insulin, B1-4 insulin, AspB28 human insulin (insulin aspart), LysB28/ProB29 human insulin (insulin lispro), LysB03/GluB29 human insulin (insulin glulisine) or GlyA21/ArgB31/ArgB32 human insulin (insulin glargine). The only proviso of an insulin analogue is that it has a sufficient insulin activity. An overview of the structure-activity relationships for insulin analogues, with discussion of which amino acid exchanges, deletions and/or additions are tolerated is provided in Mayer, J. P. et al. (2007; supra). The insulin analogues are preferably such wherein one or more of the naturally occurring amino acid residues, preferably one, two or three of them, have been substituted by another amino acid residue. Further examples of insulin analogues are C-terminal truncated derivatives such as des(B30) human insulin; B-chain N-terminal truncated insulin analogues such as des PheB1 insulin or des B1-4 insulin; insulin analogues wherein the A-chain and/or B-chain have an N-terminal extension, including so-called "pre-insulins" where the B-chain has an N-terminal extension; and insulin analogues wherein the A-chain and/or the B-chain have C-terminal extension. For example one or two Arg may be added to position B1. Examples of insulin analogues are described in the following patents and equivalents thereto: US 5,618,913, EP 0 254 516 A2 and EP 0 280 534 A2. An overview of insulin analogues in clinical use is provided in Mayer J. P. et al. (2007, supra). Insulin analogues or their precursors are typically prepared using gene technology techniques well known to those skilled in the art, typically in bacteria or yeast, with subsequent enzymatic or synthetic manipulation if required. Alternatively, insulin analogues can be prepared chemically (Cao, Q. P. et al. (1986) Biol. Chem. Hoppe Seyler, 367, 135-140, No. 2). Examples of specific insulin analogues are insulin aspart (i.e. AspB28 human insulin); insulin lispro (i.e. LysB28, ProB29 human insulin); insulin glulisine (ie. LysB03, GluB29 human insulin); and insulin glargine (i.e. GlyA21, ArgB31, ArgB32 human insulin).

Exemplary DPP-4 Inhibitors are
sitagliptin: (*R*)-4-oxo-4-[3-(trifluoromethyl)-5,6-dihydro[1,2,4]triazolo[4,3-*a*]-pyrazin-7(8*H*)-yl]-1-(2,4,5-trifluorophenyl)butan-2-amine,
vildagliptin: (*S*)-1-[*N*-(3-hydroxy-1-adamantyl)glycyl]pyrrolidine-2-carbonitrile,
saxagliptin: (1*S*,3*S*,5*S*)-2-[(2*S*)-2-amino-2-(3-hydroxy-1-adamantyl)-acetyl]-2-azabicyclo[3.1.0]hexane-3-carbonitrile,
linagliptin 8-[(3*R*)-3-aminopiperidin-1-yl]-7-(but-2-yn-1-yl)-3-methyl-1-[(4-methyl-quinazolin-2-yl)methyl]-3,7-dihydro-1*H*-purine-2,6-dione) adogliptin (2-({6-[(3*R*)-3-aminopiperidin-1-yl]-3-methyl-2,4-dioxo-3,4-dihydropyrimidin-1(2*H*)-yl}methyl)-benzonitrile, and
berberine which is a quaternary ammonium salt from the group of isoquinoline alkaloids found in in the roots, rhizomes, stems, and bark of plants such as *Berberis,* goldenseal (*Hydrastis canadensis*), and *Coptis chinensis.*

The individual compounds of the pharmaceutical composition of the present invention can be combined in one formulation or contained in several formulations for e.g. simultaneous or subsequent, i.e. sequential administration(s), or combinations thereof.

According to the present invention the combination of at least one FGF-21 compound and at least one GLP-1R agonist, surprisingly resulted in a synergistic effect in lowering plasma glucose levels as shown with the animal models in the Examples. The animal models are an ob/ob or obese mouse and a db/db mouse. The ob/ob mouse is a mutant mouse which cannot produce the hormone leptin which regulates the appetite. Consequently, the ob/ob mouse eats excessively and becomes profoundly obese. It is a standard animal model for hyperglycemia, insulin resistance and obesity. Another standard animal model for diabetes is the db/db mouse carrying a deficient leptin receptor activity. Also this mouse is characterized by obesity, hyperglycemia and insulin resistance.

The pharmaceutical composition of the present invention comprises therapeutically effective amounts of the individual compounds and generally an acceptable pharmaceutical carrier, diluent or excipient, e.g. sterile water, physiological saline, bacteriostatic saline, i.e. saline containing about 0.9% mg/ml benzyl alcohol, phosphate-buffered saline, Hank's solution, Ringer's-lactate, lactose, dextrose, sucrose, trehalose, sorbitol, Mannitol, and the like. The composition is generally a solution or suspension. It can be administered orally, subcutaneously, intramuscularly, pulmonary, by inhalation and/or through sustained release administrations. Preferably, the composition is administered subcutaneously.

The term "therapeutically effective amount" generally means the quantity of a compound that results in the desired therapeutic and/or prophylactic effect without causing unacceptable side-effects. A typical dosage range is from about 0.01 mg per day to about 1000 mg per day. A preferred dosage range for each therapeutically effective compound is from about 0.1 mg per day to about 100 mg per day and a most preferred dosage range is from about 1.0 mg/day to about 10 mg/day, in particular about 1-5 mg/day.

In case of subsequent administration(s), the individual compounds of the pharmaceutical composition are administered during a time period where the synergistic effect of the FGF-21 compound and the GLP-1R agonist are still measurable e.g. in a "glucose tolerance test", as e.g. shown in the Examples. The glucose tolerance test is a test to determine how quickly glucose is cleared from the blood after administration of glucose. The glucose is most often given orally ("oral glucose tolerance test" or "OGTT"). The time period for the subsequent administration of the individual compounds, in particular of the FGF-21 compound and the GLP-1R agonist, is usually within one hour, preferably, within half an hour, most preferably within 15 minutes, in particular within 5 minutes.

Generally, the application of the pharmaceutical composition to a patient is one or several times per day, or one or several times a week, or even during longer time periods as the case may be. The most preferred application of the pharmaceutical composition of the present invention is a subcutaneous application one to three times per day in a combined dose.

The pharmaceutical composition of the present invention lowers blood glucose levels up to normo-glycaemic levels and increases energy expenditure by faster and more efficient glucose utilization, and thus is useful for treating Type 2 diabetes.

Consequently, the present invention is also directed to the use of the described pharmaceutical composition(s) for the preparation of a medicament for treating Type 2 diabetes in a patient, as defined in claim 12. Also disclosed is a method for treating Type 2 diabetes in a patient. According to the present invention, the patient is a sulfonylurea-treated Type 2-diabetic patient. The medicament can be prepared by methods known to a person skilled in the art, e.g. by mixing the pharmaceutically effective amounts of the compound or compounds with an acceptable pharmaceutical carrier, diluent or excipient, as described above.

The following figures and examples are for the purpose of illustration only and are not intended to be limiting of the present invention.

### Figures

- Fig. 1: shows the chemical structure of liraglutide.
- Fig. 2: shows the chemical structure of CJC-1131.
- Fig. 3: shows the results of an oral glucose tolerance test (OGTT) after ten days subcutaneous injection of FGF-21 together with AVE0010 in ob/ob mice.
- Fig. 4: shows the plasma glucose levels over time after subcutaneous injection of FGF-21 together with AVE0010 in ob/ob mice.
- Fig. 5: shows the results of an OGTT after twenty-one days subcutaneous injection of FGF-21 together with AVE0010 in db/db mice.
- Fig. 6: shows the plasma glucose levels over time after subcutaneous injection of FGF-21 together with AVE0010 in db/db mice.

### Examples

### 1. Treatment of ob/ob mice

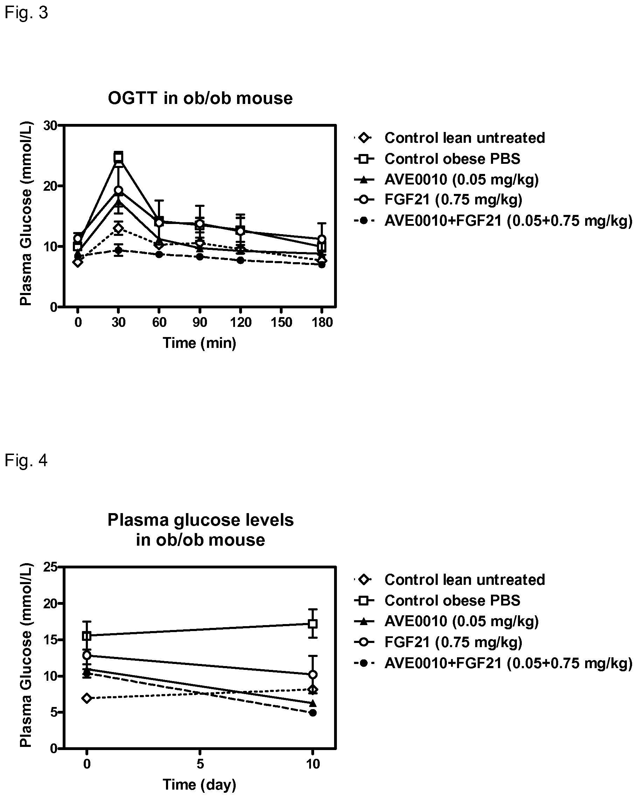

Female *ob*/*ob* mice (B6.V-LEP OB/J, age of 6 weeks) were obtained from Charles Rivers Laboratories (Sulzfeld, Germany). Mice were randomly assigned to treatment or vehicle groups, and the randomization was stratified by body weight and fed blood glucose levels. The animals were housed in groups of 6 at 23°C and on a 12 h light-dark cycle. All experimental procedures were conducted according to German Animal Protection Law.
*Ob*/*ob* mice were treated with vehicle (PBS), 0.05 mg · kg⁻¹ · day⁻¹ AVE0010 (SEQ ID NO:9), 0.75 mg · kg⁻¹ · day⁻¹ recombinant human FGF-21 (SEQ ID NO: 2) or a combined dose of FGF-21 (SEQ ID NO: 2) and AVE0010 (SEQ ID NO:9), (0.75 + 0.05 mg · kg⁻¹ · day⁻¹) subcutaneously once daily. Mice were fed *ad libitum* with standard rodent chow during the drug treatment periods. Body weight was recorded every other day, and food intake was measured once a week throughout the study. One day before the first treatment and at study day 10 blood glucose was measured by tail tip bleeding under fed conditions. As shown in Figure 4 the blood glucose levels of the treated mice became normo-glycaemic. On study day 8 a glucose tolerance test (OGTT) was performed. Fasted mice were orally challenged with 2 g · kg⁻¹ glucose. Blood glucose was measured at indicated time points by tail tip bleeding without anaesthesia. The results of the OGTT are shown in Figure 3. Compared to the administration of only FGF-21 or only AVE0010 glucose tolerance was markedly stronger improved by combination treatment. The combination treated obese animals were even more glucose tolerant than lean control animals.

### 2. Treatment of db/db mice

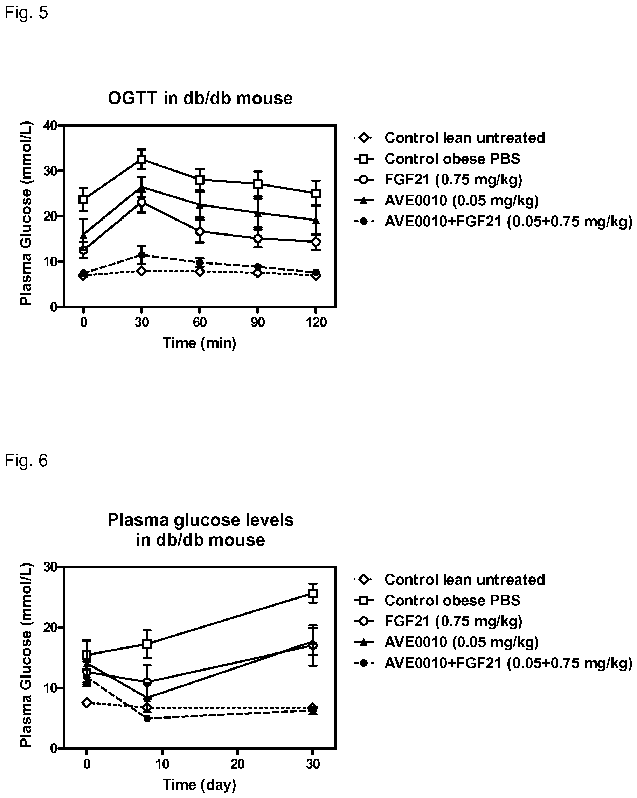

Female *db*/*db* mice (BKS.Cg-m +/+ Leprdb /J, age of 6 weeks) were treated with vehicle (PBS), 0.05 mg · kg⁻¹ · day⁻¹ AVE0010, 0.75 mg · kg⁻¹ · day⁻¹ recombinant human FGF-21 (SEQ ID NO: 2) or a combined dose of FGF-21 (SEQ ID NO: 2) and AVE0010 (SEQ ID NO:9), (0.75 + 0.05 mg · kg⁻¹ · day⁻¹) subcutaneously once daily. Mice were fed ad *libitum.* Before the first treatment, after one week and 4 weeks blood glucose and HbA1c were measured under fed conditions. After 21 days of treatment an oral glucose tolerance test (OGTT) was initiated. Fasted mice were orally challenged with 2 g · kg⁻¹ glucose solution and blood glucose was measured at indicated time points. The results are shown in Figure 6 and 7. The administration of the FGF21 plus AVE0010 combination results in normalisation of blood glucose and improved dramatically the glucose tolerance compared to the vehicle treated obese control. On the other hand leads the single treatment of FGF21 or AVE0010 compared to the combination only to inhibition of blood glucose increase and a small improvement in glucose tolerance.

### Sequences

Human FGF-21 (SEQ ID NO: 1):
Mutated FGF-21 (G + FGF21 H29-S209; SEQ ID NO: 2):
Human GLP-1 (7-37) (SEQ ID NO: 3):
   HAEGTFTSDVSSYLEGQAAKEFIAWLVKGRG-NH₂
Human GLP-1(7-36)NH₂ (SEQ ID NO: 4):
   HAEGTFTSDVSSYLEGQAAKEFIAWLVKGR-NH₂
Exendin-4 (SEQ ID NO: 5):
   HGEGTFTSDLSKQMEEEAVRLFIEWLKNGGPSSGAPPPS-NH₂
Exenatide (SEQ ID NO: 6):
   HGEGTFTSDLSKQMEEEAVRLFIETLKNGGPSSGAPPPS-NH₂
Oxyntomodulin (SEQ ID NO: 7):
   HSQGTFTSDYSKYLDSRRAQDFVQWLMNTKRNRNNIA-NH₂
Lixisenatide (SEQ ID NO: 8)
   HGEGTFTSDLSKQMEEEAVRLFIEWLKNGGPSSGAPPSKKKKKK-NH2
*AVE0010* (SEQ ID NO: 9):
   HGEGTFTSDLSKQMEEEAVRLFIEWLKNGGPSSGAPPSKKKKKK-NH2

## Claims

1. A pharmaceutical composition containing at least one FGF-21 (fibroblast growth factor 21) compound and at least one GLP-1 R (glucagon-like peptide-1 receptor) agonist for use in treating Type 2-diabetes in a patient, wherein the patient is a sulfonylurea-treated Type 2-diabetic patient.

2. The pharmaceutical composition for use of claim 1, wherein the composition further contains at least one anti-diabetic drug and/or at least one DPP-4 (dipeptidyl peptidase-4) inhibitor.

3. The pharmaceutical composition for use of claim 1 or 2, wherein the FGF-21 compound(s), the GLP-1 R agonist(s), optionally the anti-diabetic drug(s) and optionally the DPP-4 inhibitor are combined in one formulation or contained in several formulations.

4. The pharmaceutical composition for use of claim 3, wherein the formulations of the FGF-21 compound(s), the GLP-1 R agonist(s), optionally the anti-diabetic drug(s) and optionally the DPP-4 inhibitor are suitable for simultaneous or subsequent administration(s).

5. The pharmaceutical composition for use of at least one of the claims 1-4, wherein the FGF-21 compound is selected from FGF-21 or a FGF-21 mimetic.

6. The pharmaceutical composition for use of claim 5, wherein the FGF-21 mimetic is selected from a protein having at least about 96% amino acid sequence identity to the amino acid sequence shown in SEQ ID NO: 1 and having FGF-21 activity, a FGF-21 fusion protein and/or a FGF-21 conjugate.

7. The pharmaceutical composition for use of claim 6, wherein the FGF-21 mimetic is selected from a FGF-21 mutein, a FGF-21-Fc fusion protein, a FGF-21-HSA fusion protein and/or a PEGylated FGF-21.

8. The pharmaceutical composition for use of at least one of the claims 1-7, wherein the GLP-1 R agonist is selected from a bioactive GLP-1, a GLP-1 analog or a GLP-1 substitute.

9. The pharmaceutical composition for use of claim 8, wherein the GLP-1 R agonist is selected from GLP-1 (7-37), GLP-1 (7-36)amide, exendin-4, liraglutide, CJC-1131, albugon, albiglutide, exenatide, exenatide-LAR, oxyntomodulin, lixisenatide, geniproside, a short peptide with GLP-1 R agonistic activity and/or a small organic compound with GLP-1 R agonistic activity.

10. The pharmaceutical composition for use of at least one of the claims 2-9, wherein the anti-diabetic drug is selected from metformin, a thiazolidinedione, a sulphonylurea, and/or insulin.

11. The pharmaceutical composition for use of at least one of the claims 2-10, wherein the DPP-4 inhibitor is selected from sitagliptin, vildagliptin, saxagliptin, linagliptin, adogliptin and/or berberine.

12. Use of a pharmaceutical composition as defined in at least one of the claims 1-11 for the preparation of a medicament for treating Type 2-diabetes in a patient, wherein the patient is a sulfonylurea-treated Type 2-diabetic patient.

## Patentansprüche

1. Pharmazeutische Zusammensetzung, enthaltend wenigstens eine FGF-21(Fibroblast Growth Factor 21)-Verbindung und wenigstens einen GLP-1R(Glucagon-like Peptide-1 Receptor)-Agonisten zur Verwendung bei der Behandlung von Typ-2-Diabetes bei einem Patienten, wobei es sich bei dem Patienten um einen mit Sulfonylharnstoff behandelten Patienten mit Typ-2-Diabetes handelt.

2. Pharmazeutische Zusammensetzung zur Verwendung nach Anspruch 1, wobei die Zusammensetzung ferner wenigstens ein Antidiabetikum und/oder wenigstens einen DPP-4(Dipeptidyl-Peptidase-4)-Inhibitor enthält.

3. Pharmazeutische Zusammensetzung zur Verwendung nach Anspruch 1 oder 2, wobei die FGF-21-Verbindung bzw. die FGF-21-Verbindungen, der GLP-1R-Agonist bzw. die GLP-1R-Agonisten, gegebenenfalls das Antidiabetikum bzw. die Antidiabetika und gegebenenfalls der DPP-4-Inhibitor in einer Formulierung kombiniert oder in mehreren Formulierungen enthalten sind.

4. Pharmazeutische Zusammensetzung zur Verwendung nach Anspruch 3, wobei sich die Formulierungen der FGF-21-Verbindung bzw. der FGF-21-Verbindungen, des bzw. der GLP-1R-Agonisten, gegebenenfalls des Antidiabetikums bzw. der Antidiabetika und gegebenenfalls des DPP-4-Inhibitors für die gleichzeitige Verabreichung oder nacheinander erfolgende Verabreichungen eignen.

5. Pharmazeutische Zusammensetzung zur Verwendung nach wenigstens einem der Ansprüche 1-4, wobei die FGF-21-Verbindung aus FGF-21 oder einem FGF-21-Mimetikum ausgewählt ist.

6. Pharmazeutische Zusammensetzung zur Verwendung nach Anspruch 5, wobei das FGF-21-Mimetikum aus einem Protein mit mindestens etwa 96% Aminosäuresequenzidentität mit der in SEQ ID NO: 1 gezeigten Aminosäuresequenz und mit FGF-21-Aktivität, einem FGF-21-Fusionsprotein und/oder einem FGF-21-Konjugat ausgewählt ist.

7. Pharmazeutische Zusammensetzung zur Verwendung nach Anspruch 6, wobei das FGF-21-Mimetikum aus einem FGF-21-Mutein, einem FGF-21-Fc-Fusionsprotein, einem FGF-21-HSA-Fusionsprotein und/oder einem PEGylierten FGF-21 ausgewählt ist.

8. Pharmazeutische Zusammensetzung zur Verwendung nach wenigstens einem der Ansprüche 1-7, wobei der GLP-1R-Agonist aus einem bioaktiven GLP-1, einem GLP-1-Analog oder einem GLP-1-Ersatz ausgewählt ist.

9. Pharmazeutische Zusammensetzung zur Verwendung nach Anspruch 8, wobei der GLP-1R-Agonist aus GLP-1(7-37), GLP-1(7-36)amid, Exendin-4, Liraglutid, CJC-1131, Albugon, Albiglutid, Exenatid, Exenatid-LAR, Oxyntomodulin, Lixisenatid, Geniprosid, einem kurzen Peptid mit GLP-1R-Agonist-Aktivität und/oder einer kleinen organischen Verbindung mit GLP-1R-Agonist-Aktivität ausgewählt ist.

10. Pharmazeutische Zusammensetzung zur Verwendung nach wenigstens einem der Ansprüche 2-9, wobei das Antidiabetikum aus Metformin, einem Thiazolidindion, einem Sulfonylharnstoff und/oder Insulin ausgewählt ist.

11. Pharmazeutische Zusammensetzung zur Verwendung nach wenigstens einem der Ansprüche 2-10, wobei der DPP-4-Inhibitor aus Sitagliptin, Vildagliptin, Saxagliptin, Linagliptin, Adogliptin und/oder Berberin ausgewählt ist.

12. Verwendung einer pharmazeutischen Zusammensetzung mit der in wenigstens einem der Ansprüche 1-11 angegebenen Bedeutung zur Herstellung eines Arzneimittels für die Behandlung von Typ-2-Diabetes bei einem Patienten, wobei es sich bei dem Patienten um einen mit Sulfonylharnstoff behandelten Patienten mit Typ-2-Diabetes handelt.

## Revendications

1. Composition pharmaceutique contenant au moins un composé FGF-21 (facteur de croissance des fibroblastes 21) et au moins un agoniste de GLP-1R (récepteur de glucagon-like peptide-1) pour utilisation dans le traitement du diabète de type 2 chez un patient, dans laquelle le patient est un patient diabétique de type 2 traité par sulfonylurée.

2. Composition pharmaceutique pour utilisation selon la revendication 1, la composition contenant en outre au moins un médicament antidiabétique et/ou au moins un inhibiteur de DPP-4 (dipeptidyle peptidase-4).

3. Composition pharmaceutique pour utilisation selon la revendication 1 ou 2, le(s) composé(s) FGF-21, l'agoniste/les agonistes de GLP-1R, facultativement le(s) médicament(s) antidiabétique(s) et facultativement l'inhibiteur de DPP-4 étant combinés dans une formulation ou contenus dans plusieurs formulations.

4. Composition pharmaceutique pour utilisation selon la revendication 3, dans laquelle les formulations du/des composé(s) FGF-21, du/des agoniste(s) de GLP-1R, facultativement du/des médicament(s) antidiabétique(s) et facultativement de l'inhibiteur de DPP-4 sont adaptées pour administration(s) simultanée(s) ou successive(s).

5. Composition pharmaceutique pour utilisation selon au moins une des revendications 1 à 4, dans laquelle le composé FGF-21 est choisi parmi FGF-21 ou un mimétique de FGF-21.

6. Composition pharmaceutique pour utilisation selon la revendication 5, le mimétique de FGF-21 étant choisi parmi une protéine ayant au moins environ 96 % d'identité de séquence d'acides aminés avec la séquence d'acides aminés décrite dans SEQ ID NO: 1 et ayant une activité FGF-21, une protéine de fusion de FGF-21 et/ou un conjugué de FGF-21.

7. Composition pharmaceutique pour utilisation selon la revendication 6, dans laquelle le mimétique de FGF-21 est choisi parmi une mutéine de FGF-21, une protéine de fusion de FGF-21-Fc, une protéine de fusion de FGF-21-HSA et/ou un FGF-21 PEGylé.

8. Composition pharmaceutique pour utilisation selon au moins une des revendications 1 à 7, dans laquelle l'agoniste de GLP-1R est choisi parmi un GLP-1 bioactif, un analogue de GLP-1 ou un substitut de GLP-1.

9. Composition pharmaceutique pour utilisation selon la revendication 8, dans laquelle l'agoniste de GLP-1R est choisi parmi GLP-1(7-37), GLP-1(7-36)amide, exendin-4, liraglutide, CJC-1131, albugon, albiglutide, exénatide, exénatide-LAR, oxyntomoduline, lixisénatide, géniproside, un peptide court ayant une activité agoniste de GLP-1R et/ou un petit composé organique ayant une activité agoniste de GLP-1R.

10. Composition pharmaceutique pour utilisation selon au moins une des revendications 2 à 9, dans laquelle le médicament antidiabétique est choisi parmi la metformine, une thiazolidinedione, une sulfonylurée, et/ou l'insuline.

11. Composition pharmaceutique pour utilisation selon au moins une des revendications 2 à 10, dans laquelle l'inhibiteur de DPP-4 est choisi parmi la sitagliptine, la vildagliptine, la saxagliptine, la linagliptine, l'adogliptine et/ou la berbérine.

12. Utilisation d'une composition pharmaceutique comme définie dans au moins une des revendications 1 à 11 pour la préparation d'un médicament pour traiter le diabète de type 2 chez un patient, dans laquelle le patient est un patient diabétique de type 2 traité par sulfonylurée.


### SEQUENCE LISTING

<110> Sanofi
<120> Pharmaceutical composition for treating a metabolic syndrome
<130> 589-169 PCTEPT4
<150> EP 10305070.4
   <151> 2010-01-21
<150> PCT/EP2011/050793
   <151> 2011-01-21
<150> EP 14163127.5
   <151> 2011-01-21
<150> EP 16 205 881.2
   <151> 2011-01-21
<160> 9
<170> PatentIn version 3.3
<210> 1
   <211> 209
   <212> PRT
   <213> Homo sapiens
<400> 1
   Met Asp Ser Asp Glu Thr Gly Phe Glu His Ser Gly Leu Trp Val Ser
   1               5                   10                  15
   Val Leu Ala Gly Leu Leu Leu Gly Ala Cys Gln Ala His Pro Ile Pro
               20                  25                  30
   Asp Ser Ser Pro Leu Leu Gln Pro Gly Gly Gln Val Arg Gln Arg Tyr
           35                  40                  45
   Leu Tyr Thr Asp Asp Ala Gln Gln Thr Glu Ala His Leu Glu Ile Arg
       50                  55                  60
   Glu Asp Gly Thr Val Gly Gly Ala Ala Asp Gln Ser Pro Glu Ser Leu
   65                  70                  75                  80
   Leu Gln Leu Lys Ala Leu Lys Pro Gly Val Ile Gln Ile Leu Gly Val
                   85                  90                  95
   Lys Thr Ser Arg Phe Leu Cys Gln Arg Pro Asp Gly Ala Leu Tyr Gly
               100                 105                 110
   Ser Leu His Phe Asp Pro Glu Ala Cys Ser Phe Arg Glu Leu Leu Leu
           115                 120                 125
   Glu Asp Gly Tyr Asn Val Tyr Gln Ser Glu Ala His Gly Leu Pro Leu
       130                 135                 140
   His Leu Pro Gly Asn Lys Ser Pro His Arg Asp Pro Ala Pro Arg Gly
   145                 150                 155                 160
   Pro Ala Arg Phe Leu Pro Leu Pro Gly Leu Pro Pro Ala Pro Pro Glu
                   165                 170                 175
   Pro Pro Gly Ile Leu Ala Pro Gln Pro Pro Asp Val Gly Ser Ser Asp
               180                 185                 190
   Pro Leu Ser Met Val Gly Pro Ser Gln Gly Arg Ser Pro Ser Tyr Ala
           195                 200                 205
   Ser
<210> 2
   <211> 182
   <212> PRT
   <213> unknown
<220>
   <223> G linked to fragment of human FGF21 from H29 to S209
<400> 2
   Gly His Pro Ile Pro Asp Ser Ser Pro Leu Leu Gln Phe Gly Gly Gln
   1               5                   10                  15
   Val Arg Gln Arg Tyr Leu Tyr Thr Asp Asp Ala Gln Gln Thr Glu Ala
               20                  25                  30
   His Leu Glu Ile Arg Glu Asp Gly Thr Val Gly Gly Ala Ala Asp Gln
           35                  40                  45
   Ser Pro Glu Ser Leu Leu Gln Leu Lys Ala Leu Lys Pro Gly Val Ile
       50                  55                  60
   Gln Ile Leu Gly Val Lys Thr Ser Arg Phe Leu Cys Gln Arg Pro Asp
   65                  70                  75                  80
   Gly Ala Leu Tyr Gly Ser Leu His Phe Asp Pro Glu Ala Cys Ser Phe
                   85                  90                  95
   Arg Glu Leu Leu Leu Glu Asp Gly Tyr Asn Val Tyr Gln Ser Glu Ala
               100                 105                 110
   His Gly Leu Pro Leu His Leu Pro Gly Asn Lys Ser Pro His Arg Asp
           115                 120                 125
   Pro Ala Pro Arg Gly Pro Ala Arg Phe Leu Pro Leu Pro Gly Leu Pro
       130                 135                 140
   Pro Ala Pro Pro Glu Pro Pro Gly Ile Leu Ala Pro Gln Pro Pro Asp
   145                 150                 155                 160
   Val Gly Ser Ser Asp Pro Leu Ser Met Val Gly Pro Ser Gln Gly Arg
                   165                 170                 175
   Ser Pro Ser Tyr Ala Ser
               180
<210> 3
   <211> 31
   <212> PRT
   <213> unknown
<220>
   <223> Fragment of human GLP-1 (7 to 37)
<220>
   <221> MOD_RES
   <222> (31)..(31)
   <223> AMIDATION
<400> 3
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 4
   <211> 30
   <212> PRT
   <213> unknown
<220>
   <223> fragment of human GLP-1 (7-36)
<220>
   <221> MOD_RES
   <222> (30)..(30)
   <223> AMIDATION
<400> 4
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 5
   <211> 39
   <212> PRT
   <213> Heloderma suspectum
<220>
   <221> MOD_RES
   <222> (39)..(39)
   <223> AMIDATION
<400> 5
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 6
   <211> 39
   <212> PRT
   <213> Heloderma suspectum
<220>
   <221> MOD_RES
   <222> (39)..(39)
   <223> AMIDATION
<400> 6
   His Gly Glu Gly Thr Phe Thr Ser Asp Leu Ser Lys Gln Met Glu Glu
   1               5                   10                  15
   Glu Ala Val Arg Leu Phe Ile Glu Thr Leu Lys Asn Gly Gly Pro Ser
               20                  25                  30
   Ser Gly Ala Pro Pro Pro Ser
           35
<210> 7
   <211> 37
   <212> PRT
   <213> Homo sapiens
<220>
   <221> MOD_RES
   <222> (37)..(37)
   <223> AMIDATION
<400> 7
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asn Thr Lys Arg Asn
               20                  25                  30
   Arg Asn Asn Ile Ala
           35
<210> 8
   <211> 44
   <212> PRT
   <213> unknown
<220>
   <223> GLP-1 like peptide
<220>
   <221> MOD_RES
   <222> (44)..(44)
<400> 8
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 9
   <211> 44
   <212> PRT
   <213> unknown
<220>
   <223> GLP-1 like peptide
<220>
   <221> MOD_RES
   <222> (44)..(44)
   <223> AMIDATION
<400> 9
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40